# FASCICULE DE BREVET EUROPEEN

(11) **EP 3 784 319 B1**
(45) Date de publication et mention de la délivrance du brevet: **06.12.2023**
(21) Numéro de dépôt: 18800705.8
(22) Date de dépôt: 19.10.2018
(51) Int. Cl.: A61M 25/00, A61M 37/00

(54) **DISPOSITIF DE TRAITEMENT D'UNE PARTIE DU CORPS HUMAIN COMPRENANT AU MOINS UNE AIGUILLE AIMANTEE**
VORRICHTUNG ZUM BEHANDELN EINES TEILS DES MENSCHLICHEN KÖRPERS MIT MINDESTENS EINER MAGNETISIERTEN NADEL
DEVICE FOR TREATING A PART OF THE HUMAN BODY COMPRISING AT LEAST ONE MAGNETISED NEEDLE

(30) Priorité: 26.04.2018 WO PCT/FR2018/051058
(43) Date de publication de la demande: 03.03.2021
(73) Titulaire: Paradis, Line, Dubai (AE)
(72) Inventeur: Paradis, Line, Dubai (AE)
(74) Mandataire: Cabinet Beau de Loménie
(86) Numéro de dépôt international: PCT/FR2018/052608
(87) Numéro de publication internationale: WO 2019/207212

(56) Documents cités:
- WO-A1-2005/110395
- WO-A1-2017/210017
- WO-A2-2007/011788
- DE-A1-102011 120 366
- KR-A- 20130 058 843

## Description

### Arrière-plan de l'invention

On dispose actuellement de différentes techniques pour effacer une trace pigmentaire de la peau ou les lèvres.

La trace pigmentaire peut être constituée par les pigments d'un tatouage et différentes méthodes de détatouage ont été développées. Parmi les techniques de détatouage existantes, on peut citer les méthodes utilisant une composition de détatouage qui dissout les pigments du tatouage et provoque leur rejet à la surface de la peau. Ces techniques fournissent de bons résultats mais il peut être souhaitable de les perfectionner davantage en diminuant le nombre de séances nécessaires pour complètement effacer le tatouage.

La réalisation d'un tatouage constitue un évènement conduisant, de manière intentionnelle, à la formation d'une trace pigmentaire. Il peut toutefois exister d'autres évènements, non intentionnels cette fois-ci, qui conduisent à une modification de l'aspect d'une partie du corps et à l'apparition d'une trace pigmentaire. C'est en particulier le cas lorsque le sujet a été exposé à la déflagration d'une charge explosive qui a conduit à une incrustation d'éclats métalliques dans la peau du sujet. C'est aussi le cas lorsque le sujet a subi un accident de la route qui peut résulter en l'incrustation de particules de bitume ou de goudron.

Il est connu d'utiliser une technique d'irradiation par laser pour effacer de telles traces pigmentaires. Toutefois, cette technique n'est pas entièrement satisfaisante car elle n'aboutit pas toujours à un effacement complet. Cette technique conduit même, dans certains cas, à une altération inesthétique supplémentaire de l'aspect de la surface traitée.

La Déposante a recherché des méthodes pour effacer efficacement une trace pigmentaire de la peau ou des lèvres sans altérer l'aspect de la surface traitée de manière notable.

On connaît KR 20130058843 et DE 102011120366 qui décrivent des dispositifs de tatouage. WO 2005/110395 et WO 2017/210017 décrivent des systèmes utilisant des particules magnétiques.

### Objet et résumé de l'invention

Le brevet décrit un premier dispositif motorisé de traitement d'une partie du corps humain, comprenant au moins :
- une partie de traitement comprenant au moins une aiguille aimantée destinée à venir en contact avec une partie du corps humain, et
- un dispositif d'entraînement motorisé relié à la partie de traitement et apte à imposer un mouvement à ladite au moins une aiguille aimantée, et lorsque ce mouvement est un mouvement de translation la partie de traitement comprend une unique aiguille aimantée ou une pluralité d'aiguilles aimantées solidarisées entre elles par soudure.

Le brevet décrit également un deuxième dispositif non-motorisé de traitement d'une partie du corps humain sous la forme d'une pièce à main, comprenant au moins :
- une partie de traitement comprenant au moins une aiguille aimantée destinée à venir en contact avec une partie du corps humain, et
- une partie de préhension.

Les premier et deuxième dispositifs décrits ci-dessus mettent en oeuvre au moins une aiguille aimantée afin de réaliser un traitement d'une partie du corps humain. La partie du corps humain peut être la peau ou les lèvres. Dans le premier dispositif, un moteur met en mouvement ladite au moins une aiguille aimantée alors que le mouvement de celle-ci est uniquement produit par manipulation par un opérateur dans le deuxième dispositif. Le deuxième dispositif est dépourvu de moteur et de dispositif d'entraînement.

L'invention concerne un procédé de traitement cosmétique de la peau ou des lèvres selon la revendication 1.

Le dispositif décrit permet d'effacer efficacement une trace pigmentaire de la peau ou des lèvres sans altérer l'aspect de la surface traitée de manière notable. En particulier, la Déposante a constaté que les dispositifs décrits ci-dessus sont utiles pour extraire un composé métallique incrusté en utilisant l'effet d'attraction magnétique de ce composé par ladite au moins une aiguille aimantée. Ainsi, ces dispositifs sont utilisés pour du détatouage.

Dans un exemple de réalisation, ladite au moins une aiguille aimantée est formée d'un matériau choisi parmi : l'acier inoxydable, le titane, les alliages de titane, ou le nitrure de titane.

Dans un exemple de réalisation, le diamètre de ladite au moins une aiguille aimantée peut inférieur ou égal à 0,40 mm, par exemple compris entre 0,10 mm et 0,40 mm.

La ou les aiguilles aimantées peuvent être animées de différents types de mouvements lors de l'utilisation du dispositif.

En particulier, le dispositif est motorisé et le dispositif d'entraînement peut être apte à imposer un mouvement de translation à ladite au moins une aiguille aimantée. En particulier, le dispositif d'entraînement peut être apte à imposer un mouvement de va et vient à ladite au moins une aiguille aimantée.

Selon un autre exemple, la partie de traitement est sous la forme d'un rouleau mobile en rotation autour de son axe et portant ladite au moins une aiguille aimantée.

Dans un exemple de réalisation, la partie de traitement comprend une unique aiguille aimantée.

Il est encore décrit un dispositif motorisé ou non-motorisé de traitement d'une partie du corps humain, comprenant au moins :
- une partie de traitement comprenant une ou plusieurs aiguille(s) aimantée(s) destinée(s) à venir en contact avec une partie du corps humain, et
- une partie de préhension.

Les différentes caractéristiques décrites dans la présente divulgation sont applicables à cet objet.

Ladite au moins une aiguille aimantée peut présenter un champ d'induction magnétique supérieur à 100 Gauss, par exemple compris entre 100 Gauss et 6000 Gauss.

Le procédé est un procédé de détatouage de la peau ou des lèvres, et le composé est un pigment de tatouage.

Le brevet décrit encore un procédé de fabrication d'un dispositif tel que décrit plus haut, comprenant au moins :
- l'aimantation de ladite au moins une aiguille par soumission à un champ d'induction magnétique d'un aimant permanent ayant une intensité supérieure ou égale à 100 Gauss.

### Brève description des dessins

D'autres caractéristiques et avantages de l'invention ressortiront de la description suivante en référence aux dessins annexés, sur lesquels :
- la figure 1 illustre, de manière schématique et partielle, un exemple de premier dispositif (cas du dispositif motorisé),
- la figure 2 illustre, de manière schématique, un détail du dispositif de la figure 1,
- la figure 3 illustre, de manière schématique et partielle, la réalisation d'un exemple de procédé de traitement cosmétique selon l'invention utilisant le dispositif de la figure 1,
- la figure 4 illustre, de manière schématique et partielle, une variante de premier dispositif,
- la figure 5 illustre, de manière schématique et partielle, une autre variante de premier dispositif,
- la figure 6 illustre, de manière schématique et partielle, un exemple de deuxième dispositif (cas du dispositif non-motorisé) ne formant pas partie de l'invention, et
- la figure 7 illustre, de manière schématique et partielle, une variante de deuxième dispositif ne formant pas partie de l 'invention.

### Description détaillée de modes de réalisation

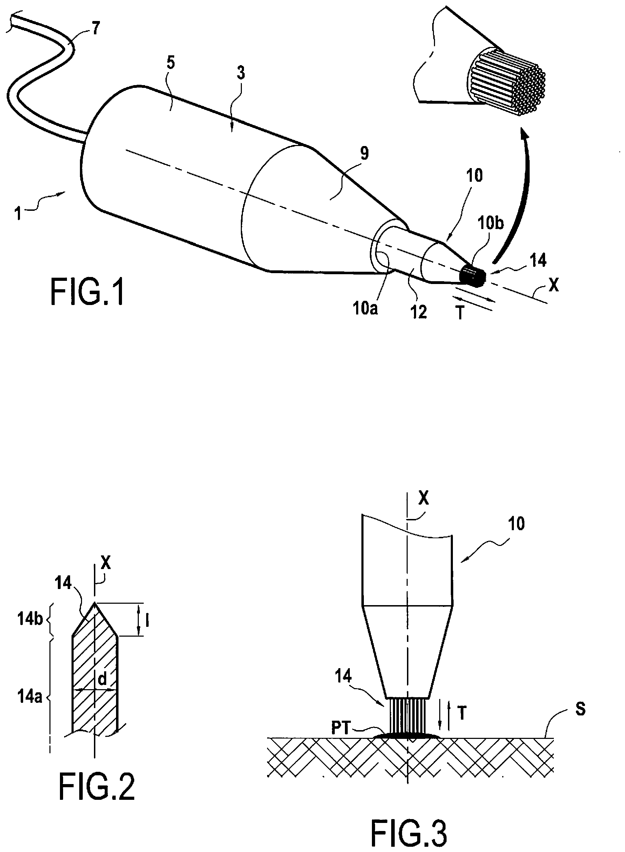

On a représenté à la figure 1 un exemple de premier dispositif 1. Le dispositif 1 se présente sous la forme d'une pièce à main. Le dispositif 1 comprend un corps 3 présentant une partie proximale 5 dans laquelle un dispositif d'entraînement en translation est logé. Le dispositif d'entraînement est configuré pour imposer un mouvement de va et vient en translation à un faisceau d'aiguilles 14. Le dispositif d'entraînement constitue un dispositif motorisé qui n'est pas original en soi. Le dispositif d'entraînement est relié à un générateur électrique (non représenté) par l'intermédiaire du câble électrique 7. Le corps 3 présente en outre une partie distale 9 formant partie de préhension. A titre d'exemple de corps 3 renfermant un dispositif d'entraînement utilisable dans le cadre de l'invention, on peut citer le dispositif commercial « Dermo Power Pen » (Linda Paradis).

Le dispositif 1 comprend, en outre, un embout 10 relié au corps 3. Cet embout 10 comprend une enveloppe 12 entourant le faisceau d'aiguilles 14. Le faisceau d'aiguilles 14 s'étend à l'intérieur de l'enveloppe 12. Les aiguilles formant le faisceau d'aiguilles 14 sont solidaires entre elles et sont ainsi maintenues dans une position prédéterminée. Les aiguilles 14 sont ici soudées entre elles. Les aiguilles 14 peuvent être soudées avec ou sans emploi d'un matériau d'apport. Le faisceau 14 s'étend le long d'un axe X longitudinal. L'ensemble constitué par l'enveloppe 12 et le faisceau d'aiguilles 14 se présente ici sous la forme d'une cartouche qui est reliée au corps 3. L'enveloppe 12 définit ainsi une portion de fixation 10a de l'embout 10 qui est insérée dans un logement ménagé dans la partie distale 9 du corps 3 afin de fixer l'embout 10 au corps 3.

Le faisceau d'aiguilles 14 forme la partie de traitement du dispositif 1. Les aiguilles 14 sont ainsi destinées à être mises en contact avec la peau ou les lèvres afin de réaliser le traitement. La partie distale 10b de l'embout 10 est définie par le faisceau d'aiguilles 14. Le faisceau d'aiguilles 14 dépasse de l'enveloppe 12.

Les aiguilles 14 sont, bien entendu, stériles avant utilisation du dispositif 1. Les aiguilles 14 sont à usage unique. Les aiguilles 14 sont aimantées. Afin d'aimanter les aiguilles, on peut utiliser un aimant permanent ayant un champ d'induction magnétique supérieur à 100 Gauss, par exemple compris entre 100 Gauss et 6000 Gauss. Les aiguilles 14 peuvent présenter un champ d'induction magnétique supérieur à 100 Gauss, par exemple compris entre 100 Gauss et 6000 Gauss.

La présente invention n'est pas limitée à l'emploi d'un nombre particulier d'aiguilles 14 dans le dispositif. Avantageusement et afin de réduire tout risque de perforation de la surface traitée, on peut utiliser un faisceau à 88 ou 132 aiguilles tel que décrit dans la demande PCT/FR2018/051058. On ne sort toutefois pas du cadre de l'invention lorsque le faisceau d'aiguilles comprend un nombre différent d'aiguilles, et en particulier moins de 88 aiguilles ou plus de 132 aiguilles. On peut même utiliser un dispositif à une seule aiguille, comme cela sera rappelé dans la suite.

Les aiguilles 14 peuvent être formées d'un matériau métallique, comme de l'acier inoxydable, du titane, un alliage de titane, comme un alliage de titane et d'or. On peut par exemple utiliser des aiguilles 14 en acier inoxydable ayant une teneur massique en nickel inférieure ou égale à 9%, par exemple comprise entre 3% et 9%. L'acier inoxydable peut appartenir aux séries 200 ou 400.

Le diamètre d de chacune des aiguilles 14 peut être supérieur ou égal à 0,10 mm. A titre d'exemple, ce diamètre d peut être compris entre 0,25 mm et 0,40 mm, et par exemple être sensiblement égal à 0,3 mm ou à 0,35 mm. Par « diamètre », on entend, sauf mention contraire, la plus grande dimension transversale mesurée perpendiculairement à l'axe X longitudinal. Comme illustré sur la figure 2, les aiguilles 14 peuvent comprendre une portion de diamètre constant 14a prolongée par une portion effilée 14b de diamètre décroissant. La portion effilée 14b définit la pointe de l'aiguille, cette portion 14b est destinée à venir en contact avec la surface à traiter. On peut utiliser des aiguilles 14 à pointe courte (aiguilles connues en soi, communément désignées par l'expression anglaise « short-taper needles »). De telles aiguilles 14 présentent une pointe 14b ayant une longueur l inférieure ou égale à 2 mm. On ne sort toutefois pas du cadre de l'invention si l'on n'utilise pas des aiguilles à pointe courte. A titre d'exemple, la longueur des aiguilles aimantées 14, mesurée d'une extrémité de l'aiguille à une autre, peut être supérieure ou égale à 1 cm, par exemple supérieure ou égale à 5 cm. On ne sort toutefois pas du cadre de l'invention lorsque des aiguilles aimantées de longueur inférieure sont utilisées.

Lors de l'actionnement du dispositif d'entraînement, les aiguilles 14 sont animées d'un mouvement de va et vient le long de l'axe X. Ce mouvement de va et vient est matérialisé par la double flèche T à la figure 1. Ce mouvement de va et vient constitue un mouvement de translation alternatif en avant et en arrière le long de l'axe X. Lorsque le dispositif d'entraînement est actionné, le faisceau d'aiguilles 14 se déplace en bloc avec un mouvement de va et vient le long de l'axe X.

Un exemple de traitement cosmétique selon l'invention mettant en oeuvre le dispositif de la figure 1 va maintenant être décrit en lien avec la figure 3.

La figure 3 illustre la mise en oeuvre d'un traitement cosmétique d'une surface S de la peau ou des lèvres. La surface S peut, en particulier, être une zone du visage ou du corps. Dans le cas particulier illustré à la figure 3, le dispositif 1 est un applicateur de produit cosmétique PT. Le dispositif 1 n'est toutefois pas forcément destiné à appliquer un produit et peut être utilisé seul, comme cela sera détaillé dans la suite.

Dans un premier temps, un produit PT de traitement cosmétique est appliqué sur le faisceau d'aiguilles 14. Ce produit PT peut être à l'état liquide. Le produit PT peut être configuré pour migrer au travers de la surface S. La pression alternative produite par le mouvement de va et vient du faisceau d'aiguilles 14 participe à faciliter cette migration. Le produit PT peut permettre de réaliser le traitement cosmétique au niveau du derme. Comme illustré à la figure 3, le dispositif 1 est utile pour réaliser un détatouage de la peau ou des lèvres. Le produit PT est une composition liquide de détatouage apte à migrer au travers de la surface S. Une telle composition liquide permet d'extraire l'encre de tatouage à l'extérieur de la surface S afin de réaliser le détatouage. A titre d'exemple de composition de détatouage utilisable, on peut citer la composition commerciale « Tattoo Remoov » ^{™} (Linda Paradis).

Afin de réaliser l'application, les aiguilles 14 imprégnées du produit PT sont animées d'un mouvement de va et vient le long de l'axe X. Le faisceau 14 se déplace transversalement par rapport à la surface traitée durant l'application. Le faisceau 14 d'aiguilles a un effet de piston qui facilite la pénétration du produit. Le mouvement de va et vient des aiguilles 14 provoque un détachement de la couche superficielle de l'épiderme « stratum corneum » et provoque une légère élévation de température de la surface traitée. Cette légère élévation provoque une légère évaporation de l'eau présente dans la peau ou les lèvres, permettant au produit PT d'être aspiré à l'intérieur de la peau ou des lèvres puisque le produit PT « Tattoo Remoov » ^{™} comprend de 35% à 40% d'eau. L'interaction du produit PT avec le pigment de tatouage conduit à une expulsion de ce dernier en dehors de la surface traitée.

L'utilisation d'aiguilles aimantées 14 permet avantageusement de rendre encore plus efficace et rapide l'extraction des pigments du tatouage en dehors de la peau ou des lèvres. En effet, ces pigments constituent des composés métalliques qui sont attirés vers les aiguilles 14 par interaction magnétique, ce qui facilite leur extraction et donc l'efficacité du détatouage. Dans cet exemple, l'interaction magnétique se superpose à l'effet physico-chimique produit par l'emploi de l'applicateur et du produit de détatouage PT afin d'optimiser l'efficacité du détatouage réalisé. La mise en oeuvre de cette méthode permet avantageusement de disposer d'une technique de détatouage particulièrement efficace nécessitant un nombre de séances réduit afin de complètement effacer la trace pigmentaire du tatouage.

On a décrit, en lien avec les figures 1 à 3, un exemple de premier dispositif (cas du dispositif motorisé). Le dispositif décrit n'est toutefois pas limité à une telle structure comme cela va être illustré dans la suite.

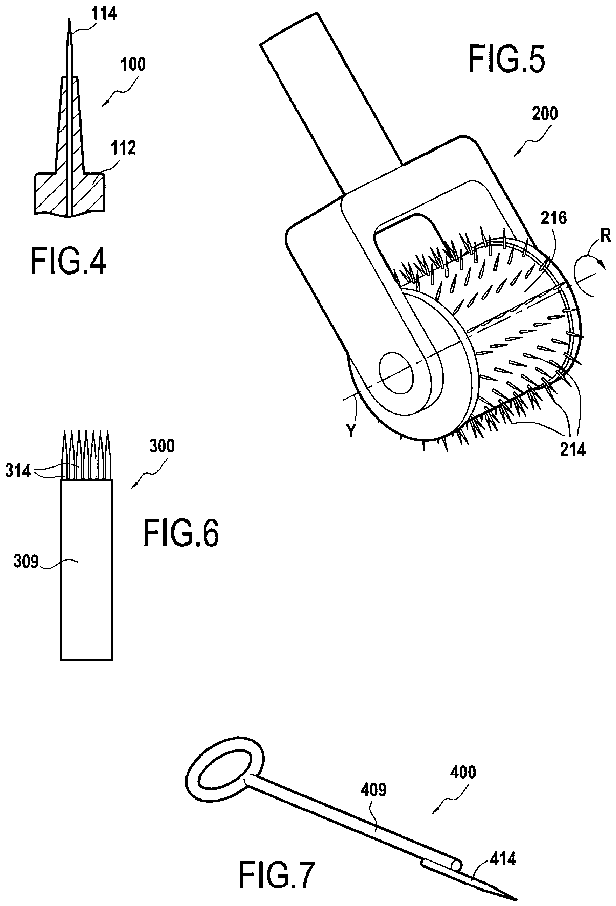

On a représenté à la figure 4 une variante de dispositif 100 motorisé lequel comprend cette fois-ci une unique aiguille aimantée 114 présente dans une enveloppe 112 et mobile en translation. On a représenté à la figure 5 une autre variante de de dispositif 200 motorisé lequel comprend une partie de traitement sous la forme d'un rouleau 216 mobile en rotation autour de son axe Y et portant une pluralité d'aiguilles aimantées 214. Le moteur est destiné à mettre en rotation le rouleau 216 et les aiguilles 214 autour de l'axe Y du rouleau. Cette rotation est matérialisée par la flèche R à la figure 5. Le dispositif 200 à rouleau de la figure 5 peut être sous la forme d'une pièce à main. On ne sort toutefois pas du cadre de l'invention si le dispositif présente une telle structure pour la partie de traitement mais est dépourvu de moteur.

Les figures 6 et 7 illustrent, quant à elles, des exemples de deuxièmes dispositifs (cas du dispositif non-motorisé) ne formant pas partie de l'invention. L'exemple de dispositif 300 de la figure 6 comprend une pluralité d'aiguilles aimantées 314 assemblées au niveau d'une même partie de préhension 309. L'exemple de dispositif 400 de la figure 7 comprend une unique aiguille aimantée 414 reliée à une partie de préhension 409. La partie de préhension 309 ou 409 peut comme illustré être distincte de la partie de traitement comprenant la ou les aiguille(s) aimantée(s) 314 ou 414. En variante, une première partie d'une aiguille aimantée peut former la partie de traitement, et une deuxième partie de cette aiguille peut former la partie de préhension.

L'expression « compris(e) entre ... et ... » doit se comprendre comme incluant les bornes.

## Revendications

1. Procédé de traitement cosmétique de la peau ou des lèvres mettant en oeuvre un dispositif (1 ; 100 ; 200) motorisé de traitement d'une partie du corps humain, le dispositif comprenant au moins :
- une partie de traitement comprenant au moins une aiguille aimantée (14 ; 114 ; 214) destinée à venir en contact avec une partie du corps humain, et
- un dispositif d'entraînement motorisé relié à la partie de traitement et apte à imposer un mouvement à ladite au moins une aiguille aimantée, et lorsque ce mouvement est un mouvement de translation (T) la partie de traitement comprend une unique aiguille aimantée ou une pluralité d'aiguilles aimantées solidarisées entre elles par soudure,
**caractérisé en ce que** le procédé est un procédé de détatouage de la peau ou des lèvres et **en ce que** il comprend au moins :
- la mise en contact de ladite au moins une aiguille aimantée avec la peau ou des lèvres, dans lequel il y a extraction d'un composé métallique incrusté dans la peau ou les lèvres en utilisant l'attraction magnétique de ce composé par ladite au moins une aiguille aimantée, le composé étant un pigment de tatouage.

2. Procédé (1 ; 100 ; 200) selon la revendication 1, dans lequel ladite au moins une aiguille aimantée (14 ; 114 ; 214) est formée d'un matériau choisi parmi : l'acier inoxydable, le titane, les alliages de titane, ou le nitrure de titane.

3. Procédé (1 ; 100) selon la revendication 1 ou selon la revendication 2, dans lequel le dispositif d'entraînement est apte à imposer un mouvement de translation (T) à ladite au moins une aiguille aimantée (14 ; 114).

4. Procédé (200) selon la revendication 1 ou 2, dans lequel la partie de traitement est sous la forme d'un rouleau (216) mobile en rotation (R) autour de son axe (Y) et portant ladite au moins une aiguille aimantée (214).

5. Procédé (100) selon l'une quelconque des revendications 1 à 4, dans lequel la partie de traitement comprend une unique aiguille aimantée (114 ; 414).

6. Procédé (100) selon l'une quelconque des revendications 1 à 5, dans lequel ladite au moins une aiguille aimantée présente un champ d'induction magnétique supérieur à 100 Gauss.

## Patentansprüche

1. Verfahren zur kosmetischen Behandlung der Haut oder der Lippen unter Verwendung einer motorisierten Vorrichtung (1; 100; 200) zur Behandlung eines Teils des menschlichen Körpers, wobei die Vorrichtung mindestens umfasst:
- einen Behandlungsabschnitt, umfassend mindestens eine magnetisierte Nadel (14; 114; 214), die dazu vorgesehen ist, mit einem Teil des menschlichen Körpers in Kontakt zu kommen, und
- eine motorisierte Antriebsvorrichtung, die mit dem Behandlungsabschnitt verbunden ist und dazu geeignet ist, die mindestens eine magnetisierte Nadel in Bewegung zu versetzen, und wobei, wenn diese Bewegung eine Translationsbewegung (T) ist, der Behandlungsabschnitt eine einzige magnetisierte Nadel oder mehrere durch Schweißen fest miteinander verbundene magnetisierte Nadeln umfasst,
**dadurch gekennzeichnet, dass** das Verfahren ein Verfahren zur Tattooentfernung für die Haut oder die Lippen ist und dass es mindestens umfasst:
- Inkontaktbringen der mindestens einen magnetisierten Nadel mit der Haut oder den Lippen, wobei eine Extraktion einer in die Haupt oder die Lippen eingebetteten Metallverbindung mittels der magnetischen Anziehung dieser Verbindung durch die mindestens eine magnetisierte Nadel stattfindet, wobei es sich bei der Verbindung um ein Tattoopigment handelt.

2. Verfahren (1; 100; 200) nach Anspruch 1, wobei die mindestens eine magnetisierte Nadel (14; 114; 214) aus einem Material ausgebildet ist, das ausgewählt ist aus: Edelstahl, Titan, Titanlegierungen oder Titannitrid.

3. Verfahren (1; 100) nach Anspruch 1 oder nach Anspruch 2, wobei die Antriebsvorrichtung dazu geeignet ist, die mindestens eine magnetisierte Nadel (14; 114) in eine Translationsbewegung (T) zu versetzen.

4. Verfahren (200) nach Anspruch 1 oder 2, wobei der Behandlungsabschnitt die Form einer Rolle (216) aufweist, die um ihre Achse (Y) drehbeweglich (R) ist und die mindestens eine magnetisierte Nadel (214) trägt.

5. Verfahren (100) nach einem der Ansprüche 1 bis 4, wobei der Behandlungsabschnitt eine einzige magnetisierte Nadel (114; 414) umfasst.

6. Verfahren (100) nach einem der Ansprüche 1 bis 5, wobei die mindestens eine magnetisierte Nadel ein magnetisches Induktionsfeld von über 100 Gauß aufweist.

## Claims

1. A cosmetic treatment method of skin or lips using a motorised device (1; 100; 200) for treating a part of a human body, the device comprising at least:
- a treatment portion comprising at least one magnetised needle (14; 114; 214) intended to come into contact with the part of the human body; and
- a motorised drive device connected to the treatment portion and capable of imposing movement upon said at least one magnetised needle, and when this movement is a translational movement (T) the treatment portion comprises a single magnetised needle or a plurality of magnetised needles secured together by welding;
**characterized in that** the method is a tattoo removal method of the skin or lips and **in that** it comprises at least:
- contacting said at least one magnetised needle with the skin or lips; wherein a metal compound embedded in the skin or lips is extracted through use of the magnetic attraction of this compound by said at least one magnetised needle, the compound being a tattoo pigment.

2. The method (1; 100; 200) according to claim 1, wherein said at least one magnetised needle (14; 114; 214) is formed of a material selected from among: stainless steel, titanium, titanium alloys or titanium nitride.

3. The method (1; 100) according to claim 1 or 2, wherein the drive device is capable of imposing a translational movement (T) upon said at least one magnetised needle (14; 114).

4. The method (200) according to claim 1 or 2, wherein said treatment portion is in the form of a roller (216) mobile in rotation (R) about a longitudinal axis (Y) of the roller and carrying said at least one magnetised needle (214).

5. The method (100) according to any one of claims 1 to 4, wherein the treatment portion comprises a single magnetised needle (114; 414).

6. The method (100) according to any one of claims 1 to 5, wherein said at least one magnetised needle has a magnetic induction field higher than 100 Gauss.
